(19) Europäisches Patentamt
European Patent Office
Office européen des brevets

(11) Veröffentlichungsnummer: **0 209 060 B1**

(12) **EUROPÄISCHE PATENTSCHRIFT**

(45) Veröffentlichungstag der Patentschrift: **21.08.91**

(51) Int. Cl.[5]: **C07C 29/10**, C07C 31/42

(21) Anmeldenummer: **86109308.6**

(22) Anmeldetag: **08.07.86**

(54) **Verfahren zur Herstellung von fluorhaltigen Diolen und Tetrolen.**

(30) Priorität: **17.07.85 DE 3525494**

(43) Veröffentlichungstag der Anmeldung:
**21.01.87 Patentblatt 87/04**

(45) Bekanntmachung des Hinweises auf die Patenterteilung:
**21.08.91 Patentblatt 91/34**

(84) Benannte Vertragsstaaten:
**BE CH DE FR GB IT LI NL**

(56) Entgegenhaltungen:
**DE-A- 1 568 378**
**FR-A- 2 235 105**
**GB-A- 794 209**

(73) Patentinhaber: **HOECHST AKTIENGESELLSCHAFT**
**Postfach 80 03 20**
**W-6230 Frankfurt am Main 80(DE)**

(72) Erfinder: **Von Werner, Konrad, Dr.**
**Buch 1 1/2**
**W-8261 Halsbach(DE)**

**EP 0 209 060 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von fluorhaltigen Diolen und Tetrolen gemäß Patentanspruch 1.

Aus DE-AS 1 568 378 ist ein Verfahren bekannt Perfluoralkylpropandiole aus den entsprechenden Perfluoralkylpropylenoxiden durch Umsetzung mit verdünnter Schwefelsäure, die zweckmäßig im Überschuß verwendet wird, herzustellen. In den Beispielen werden je 1 mol des Perfluoralkylpropylenoxids 1,6 bis 14,9 mol $H_2SO_4$ verwendet. Wie nachfolgender Vergleichsversuch A zeigt, werden hierbei nur Gemische des Perfluoralkylpropandiols mit erheblichen Mengen unerwünschter Nebenprodukte, insbesondere von Perfluoralkyletheralkoholen,erhalten.

Ein verbessertes Verfahren zur Herstellung von Perfluoralkylpropandiol ist aus JP Kokai Sho-53-084909 bekannt. Hier wird zunächst Perfluoralkylpropylenoxid mit einer Carbonsäure, beispielsweise Essigsäure, im Verhältnis 1 : 1,1 bis 1 : 1,4 in Gegenwart eines basischen Katalysators, beispielsweise Triethylamin, umgesetzt und der so gebildete Ester anschließend mit Alkohol in Gegenwart von kaustischem Alkali zum Perfluoralkylpropandiol umgewandelt. Dieses Verfahren ergibt wesentlich reinere Produkte als das zuvor genannte, ist aber als Zweistufenverfahren umständlicher durchzuführen. Beide bekannten Methoden verwenden erhebliche Mengen Säure, die entweder als solche oder neutralisiert als Salz das Abwasser belasten.

Es wurde nun ein Verfahren gefunden, das es ermöglicht, Perfluoralkylpropandiol mit sehr guten Ausbeuten und guter Reinheit in einem einstufigen Prozeß weitgehend ohne abwasserbelastende Nebenprodukte zu gewinnen.

Es handelt sich um ein Verfahren zur Herstellung von Verbindungen der Formel

$ZR_F\text{-}CH_2\text{-}CHOH\text{-}CH_2OH$ (I)

worin bedeuten:

$R_F$ einen geradkettigen,perfluorierten Alkylenrest mit 2 bis 14 C-Atomen oder in Bezug auf die Verbindung der Formel (I) verzweigten, perfluorierten Alkylenrest mit 3 bis 14 C-Atomen,

Z Fluor, Wasserstoff, Chlor oder einen Rest der Formel $-CH_2\text{-}CHOH\text{-}CH_2OH$

durch Umsetzung einer Verbindung der Formel

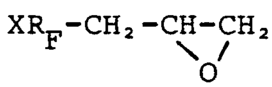

(II)

worin

$R_F$ wie oben definiert ist und

X Fluor, Wasserstoff, Chlor oder einen Rest der Formel

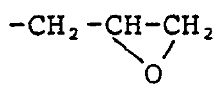

bedeutet

in Gegenwart von Wasser und einer Säure bei 20 bis 200 °C, gegebenenfalls unter Druck, dadurch gekennzeichnet, daß aus der Verbindung der Formel (II) und mindestens 1 mol Wasser je mol

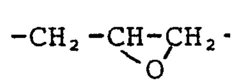

-Gruppe in der Verbindung der Formel (II) sowie mindestens einem nicht reagierenden organischen Lösungsmittel aus der Gruppe Ketone, Ether, Sulfoxide, Sulfone eine unter den Reaktionsbedingungen flüssige homogene Lösung hergestellt wird und diese Lösung umgesetzt wird, in Gegenwart von mindestens einer monomeren oder polymeren Säure, die einen $pK_S$-Wert bei 25 °C von höchstens +2 und

mindestens eine der folgenden Gruppen $-OSO_3H$;

$$-\overset{|}{\underset{|}{C}}-SO_3H;$$

-COOH aufweist, in einer Menge, die 0,001 bis 0,5 g saure H-Atome je 1 mol der Verbindung (II) enthält.

Die Verbindungen der Formel (II) können beispielsweise nach bekannten Verfahren hergestellt werden durch Umsetzung von Perfluoralkyliodiden bzw. Perfluoralkylendiiodiden mit Allylalkohol und nachfolgender Abspaltung von Iodwasserstoff. Insbesondere bei längeren Perfluoralkyl- bzw. Perfluoralkylen-Gruppen kann die C-Kette anstelle eines Fluoratoms ein Wasserstoff- oder Chloratom enthalten, bevorzugt werden jedoch solche Verbindungen eingesetzt, in denen die Kette ausschließlich C-F-Bindungen aufweist. Ferner werden bevorzugt solche Verbindungen der Formel (II) verwendet, in denen die Gruppe $R_F$ 3 bis 12, insbesondere 5 bis 11 C-Atome enthält. Verbindungen, in denen die Gruppe $R_F$ mehr als 14 C-Atome aufweist, sind schwerer zugänglich für die üblichen Anwendungen,im allgemeinen zu teuer und neigen mehr zur Bildung unerwünschter Nebenprodukte.

Die Verbindungen der Formel (II) werden in Gegenwart von Wasser und einer Säure bei 20 bis 200 °C umgesetzt. Unter 20 °C läuft die erwünschte Reaktion nur sehr langsam ab, so daß unwirtschaftliche Reaktionszeiten benötigt werden, über 200 °C nimmt die Neigung zur Bildung von Neben- und Zersetzungsprodukten erheblich zu. Vorzugsweise wird bei Temperaturen von 50 bis 150 °C gearbeitet. Bei Reaktionstemperaturen oberhalb 100 °C, bei Verwendung niedrigsiedender Lösungsmittel auch schon darunter, wird die Reaktion zweckmäßig unter dem autogenen Druck der Reaktionsmischung durchgeführt. Es kann auch zusätzlich ein Gas zur Druckerhöhung zugegeben werden. Im allgemeinen werden Drücke über 5 MPa nicht angewandt, da Apparaturen für so hohe Drücke teuer sind. Vorteilhaft wird im Bereich von normalem Atmosphärendruck bis zu 1 MPa gearbeitet.

Die Dauer der Reaktion hängt von den eingesetzten Substanzen und der angewendeten Reaktionstemperatur ab, sie beträgt etwa 0,5 bis 40 h. Vorteilhaft werden die Bedingungen so gewählt, daß eine Reaktionsdauer von 3 bis 25 h ausreicht.

Das erfindungsgemäße Verfahren besteht darin, daß unter dem Einfluß der Säure an jeden Epoxidring der Verbindung der Formel (II) 1Molekül Wasser eingeführt wird,infolgedessen ist je mol

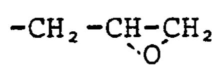

-Gruppe in der Verbindung der Formel (II) mindestens 1 mol Wasser einzusetzen, vorteilhaft wird jedoch ein Wasserüberschuß von bis zu 30 mol je mol epoxidhaltiger Gruppe in der Verbindung (II) angewandt. Mit einem Überschuß von 3 bis 15 mol Wasser je epoxidhaltige Gruppe werden gute Ergebnisse erzielt. Der Einsatz unnötig großer Wassermengen ist in der Regel unvorteilhaft, da hierdurch auch unnötig große Mengen Lösungsmittel benötigt werden, um aus der Verbindung der Formel (II) und dem Wasser eine unter den Reaktionsbedingungen flüssige homogene Lösung zu erhalten.

Das angewandte organische Lösungsmittel soll unter den Reaktionsbedingungen flüssig und mit Wasser möglichst vollständig mischbar sein, sowie eine möglichst große Menge der Verbindung der Formel (II) lösen. Es soll ferner inert sein, das heißt seinerseits nicht mit Wasser oder der Verbindung der Formel (II) unter dem Einfluß der zugesetzten Säure reagieren. Ferner sollte es bei normalem Atmosphärendruck einen Siedepunkt von 30 bis etwa 250 °C, vorzugsweise von 50 bis etwa 200 °C, aufweisen. Geeignete Lösungsmittel finden sich in der Gruppe der Ketone, beispielsweise Aceton, Methylethylketon, Dibutylketon, Cyclohexanon; der wassermischbaren Ether, beispielsweise Glykol-dimethyl- oder -diethylether, Diglykol-dimethyl- oder -diethylether, der Sulfone wie Diethylsulfon, Tetramethylensulfon; Sulfoxide wie Dimethyl- oder Diethylsulfoxid. Es können auch Gemische der obengenannten Lösungsmittel verwendet werden, vorteilhaft solche, die einen konstanten Siedepunkt aufweisen.

Vorteilhaft werden die Menge des Wassers und Art und Menge des Lösungsmittels bzw. Lösungsmittelgemisches so gewählt, daß mit diesen und der einzusetzenden Menge der Verbindung der Formel (II) bereits bei Raumtemperatur eine homogene flüssige Lösung erzielt werden kann.

Die oben beschriebene Lösung der Verbindung der Formel (II), es können auch Gemische von verschiedenen Verbindungen der Formel (II) eingesetzt werden, wird in Gegenwart von mindestens einer monomeren oder polymeren Säure, die einen $pK_S$-Wert bei 25 °C von höchstens +2 aufweist, umgesetzt. Der $pK_S$- Wert ist der negative Logarithmus der Dissoziationskonstante. "Höchstens +2" bedeutet, daß der $pK_S$- Wert auch 0 sein kann oder negative Werte annehmen kann. Die Säure soll ferner mindestens eine

der folgenden Gruppen: $-OSO_3H$,

$$-\overset{|}{\underset{|}{C}}-SO_3H,$$

-COOH aufweisen. Die freien Wertigkeiten der genannten Gruppen sind entweder mit Kohlenstoff- oder Wasserstoffatomen verbunden, im Falle der

$$-\overset{|}{\underset{|}{C}}-SO_3H\text{-Gruppe}$$

kann das C-Atom Bestandteil einer mehr oder weniger langen C-Atomkette oder eines beispielsweise aromatischen Ringsystems sein. Es kann auch Halogenatome, beispielsweise Fluor- oder Chloratome tragen. Die polymere oder monomere Säure soll keine Substituenten aufweisen, die ihrerseits reaktive Wasserstoffatome enthalten, beispielsweise $-NH_2$- bzw. OH-Gruppen, sofern letztere nicht Bestandteile der Säuregruppe selbst sind. Es können auch Mischungen von verschiedenen Säuren verwendet werden. Geeignete Säuren sind beispielsweise Alkylsulfonsäuren wie Methansulfonsäure, Arylsulfonsäuren wie Benzolsulfonsäure, Toluolsulfonsäure, polymere Sulfonsäuren wie sulfoniertes Polystyrol (z.B. die Amberlyst®-$SO_3H$-Marken der Firma Rohm und Haas), fluorierte Alkylsulfonsäuren wie Perfluorhexyl- oder Perfluoroctylsulfonsäure, polymere fluorierte Sulfonsäuren wie z.B. die Nafion®-$SO_3H$-Marken der Firma Du Pont, Trichloressigsäure, Perfluorcarbonsäuren wie Perfluoroctansäure, perfluorierte Polymere, die -COOH-Gruppen in den Seitenketten enthalten. Vorzugsweise werden unter den Reaktionsbedingungen im Reaktionsgemisch weitgehend unlösliche Säuren, insbesondere polymere Säuren, verwendet, die in einer Form vorliegen, welche eine besonders große Oberfläche aufweist. Gute Ergebnisse werden insbesondere auch mit Säuren erhalten, die eine oder mehrere Arylsulfongruppen oder eine oder mehrere, gegebenenfalls polymerisierte,Perfluoralkylgruppen aufweisen.

Die Säure bzw. die Säuren werden erfindungsgemäß in einer Menge eingesetzt, die 0,001 bis 0,5 g saurer H-Atome je 1 mol der Verbindung der Formel (II) entspricht. Unter "saure H-Atome" sind solche zu verstehen, die in Gegenwart eines großen Wasserüberschusses abdissoziieren und den sauren Charakter der jeweiligen Säure bestimmen. Unter 0,001 g saure H-Atome je mol der Verbindung (II) läuft die Reaktion zur Bildung des Perfluoralkylpropandiols nur langsam oder überhaupt nicht ab, so daß unwirtschaftlich lange Reaktionszeiten entstehen. Über 0,5 g saure H-Atome je mol der Verbindung (II) tritt in zunehmendem Maße die Bildung von unerwünschten Nebenprodukten, insbesondere von Perfluoralkyletheralkoholen ein.

Wenn die Umsetzung der Verbindung (II) mit Wasser in Gegenwart von Lösungsmitteln, wie oben beschrieben, in Gegenwart von mindestens einer polymeren Carbon- oder Sulfonsäure durchgeführt wird, so werden diese Säuren vorzugsweise in einer Menge eingesetzt, die 0,01 bis 0,5 g saure H-Atome je 1 mol der Verbindung (II) enthält. Wird andererseits die obengenannte Umsetzung in Gegenwart von mindestens einer monomeren,mindestens eine $-OSO_3H$-,

$$-\overset{|}{\underset{|}{C}}-SO_3$$

H-Gruppe aufweisenden Verbindung durchgeführt, so wird diese Verbindung vorteilhaft in einer Menge eingesetzt, die 0,002 bis 0,1 g saure H-Atome je 1 mol der Verbindung (II) enthält.

Während der Reaktion wird die Reaktionsmischung zweckmäßig durch übliche Methoden, beispielsweise Rühren oder Schütteln, in Bewegung gehalten.

Nach Beendigung der Reaktion wird die Mischung abgekühlt, gegebenenfalls entspannt und, wenn die zugegebene Säure in der Reaktionsmischung unlöslich war, diese abfiltriert, mit dem in der Reaktionsmischung verwendeten Lösungsmittel gewaschen und wiederverwendet. Die Waschflüssigkeit wird mit dem Filtrat vereinigt und zur Rückgewinnung des Lösungsmittels und Darstellung des reinen perfluoralkylpropandiols fraktioniert destilliert. Das Lösungsmittel wird ebenfalls wieder eingesetzt.

Wenn die zugegebene Säure nicht abfiltrierbar ist, werden aus der gesamten Reaktionsmischung zweckmäßig zunächst das überschüssige Wasser und das Lösungmittel fraktioniert, falls erforderlich unter Verwendung von Vakuum abdestilliert und der Rückstand mehrfach mit warmem Wasser extrahiert. Dieses Wasser enthält den wesentlichsten Teil der verwendeten Säure und kann wieder eingesetzt werden. Der Extraktionsrückstand wird im Vakuum getrocknet und anschließend durch Destillieren oder Sublimieren

unter Verwendung von Unterdruck gereinigt. Wie ersichtlich, fallen bei den beschriebenen Arten der Aufarbeitung praktisch keine Produkte an, die das Abwasser belasten oder deren Beseitung sonstige Schwierigkeiten bereiten würde. Die Reinheit der erhaltenen Perfluoralkylpropandiole ist mit der gemäß JP Kokai Sho-53-084909 mit einem Zweistufenverfahren erhaltenen Produkte vergleichbar und für die meisten Anwendungszwecke vollständig ausreichend. Die Ausbeute ist nach dem erfindungsgemäßen Verfahren verbessert.

Die nach dem neuen Verfahren hergestellten Perfluoralkylpropandiole können entweder als solche oder in Lösungen bzw. Dispersionen zur Behandlung der Oberfläche von verschiedenen Substraten, beispielsweise textilen Geweben und Gewirken, Faservliesen, Papier, Schaumstoffen, porösen Körpern usw. eingesetzt werden. Sie verleihen den behandelten Oberflächen öl- und fettabstoßende Eigenschaften, ohne sie gleichzeitig wasserabstoßend zu machen. Entsprechend behandelte Formkörper können beispielsweise dazu dienen, Gemische von Wasser und Öl in die beiden Komponenten zu trennen. Ferner dienen Perfluorpropandiole zur Herstellung von Polyestern und Polycarbonaten, die ihrerseits zur Beschichtung beispielsweise von Lichtleiterfasern dienen.

Nachfolgende Beispiele sollen die Erfindung erläutern:

Die Analyse der erzeugten Rohprodukte erfolgt durch Gelpermeations-Chromatographie (HPSEC = High Performance Size Exclusion Chromatography), die Analyse der gereinigten Produkte durch Gaschromatographie und Kernresonanz ($^1H$-; $^{19}F$- und $^{13}C$-NMR). Die OH-Zahl wurde nach üblicher Methode durch Umsetzung mit Acetanhydrid in Gegenwart katalytischer Mengen Pyridin, anschließender Hydrolyse des nicht-verbrauchten Acetanhydrids und Titration der gebildeten Essigsäure mit KOH bestimmt.

Beispiel 1

In einen 1000 $cm^3$ fassenden Glasautoklaven werden 95,2 g (0,2 mol)

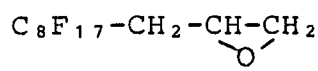

36,0 g (2,0 mol)Wasser und 350 ml Aceton gegeben. Unter Rühren und Erwärmung bildet sich eine homogene Lösung. Zu dieser werden 5,0 g Amberlyst 15® (Polystyrol mit $-SO_3H$-Seitengruppen, enthaltend 0,0205 g saure H-Atome, dies entspricht 0,102 g H je mol des eingesetzten Perfluoralkylpropylenoxids) gegeben und die Mischung 12 Stunden bei 100 °C gerührt. Nach dem Erkalten wird das im Reaktionsgemisch unlösliche Amberlyst abfiltriert und das Filtrat im Rotationsverdampfer im Vakuum bis zur Trockne eingedampft. Man erhält 96,5 g festen gelblichen Rückstands mit einem Diolgehalt von 94 % (OH-Zahl: gefunden: 212, berechnet: 227). Eine Kurzweg-Destillation des Rückstandes ergibt 89,5 g des Diols der Formel $C_8F_{17}CH_2$-CHOH-$CH_2OH$ mit einer Reinheit von mehr als 98 %, an dem folgende Werte gemessen werden: OH-Zahl 225; Kp 143 °C/1,6 kPa; Fp 114 bis 116 °C; weißer Feststoff; 90,6 % Ausbeute; typische $^{13}C$-NMR-Signale: $CH_2$ 35,5 ppm [Triplett mit $^2J$ (CF) = 20,7 Hz], (in $d_6$-Aceton, 20,15 MHz) CHOH 66,5 ppm, $CH_2OH$ 66,9 ppm.

Beispiel 2

In einen 1000 $cm^3$ Glasautoklaven werden 49,4 g (0,22 mol) $(CF_3)_2$

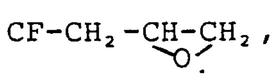

380 g 1.4-Dioxan und 43,5 g (2,42 mol) Wasser gegeben. Unter Rühren und Erwärmen bildet sich eine homogene Lösung. Zu dieser werden 6,0 g eines gekörnten perfluorierten Polymeren mit -COOH-Gruppen in den Seitenketten, enthaltend 0,025 g saure H-Atome (entsprechend 0,113 g saurer H-Atome je mol des perfluorierten Propylenoxids) gegeben und die Mischung während 13 Stunden bei 100 °C gerührt. Nach Erkalten wird der in der Reaktionsmischung unlösliche Katalysator abfiltriert, das Filtrat wird mit 500 ml einer wäßrigen Kochsalzlösung verdünnt, wobei sich zwei Phasen bilden, die voneinander getrennt werden. Die Wasser und Dioxan enthaltende Phase wird zweimal mit je 50 ml Diethylether ausgeschüttelt und dieser

Ether mit der organischen Phase vereinigt, über Natriumsulfat getrocknet und unter vermindertem Druck fraktioniert destilliert. Die Hauptfraktion ergibt 47,6 g der Verbindung $(CF_3)_2CF-CH_2-CHOH-CH_2OH$ als farblose ölige Flüssigkeit, an der folgende Werte bestimmt werden: Kp 90 °C/1,33 kPa; Reinheit 98,5 %; Ausbeute 88,6 %;
$^{19}F$-NMR (in $CDCl_3$ Signale bei hohem Feld bezüglich $CF_3COOH$):
-0,8 ppm (6F), - 108 ppm (1F)
$^{13}C$-NMR: $CF_3$ 121,2 ppm (Quartett von Dubletten); CF 91,3 ppm (Dublett von Septetten); $CH_2OH$ 67,0 ppm; CHOH 66,5 ppm (Dublett); $CH_2$ 32,3 ppm (Dublett).

Beispiel 3

19,3 g (0,046 mol) der Verbindung

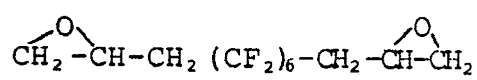

30 cm³ Tetramethylensulfon und 3,0 g (0,167 mol) Wasser werden unter Rühren erwärmt, wobei sich eine homogene Lösung bildet. Zu dieser werden 0,3 g (0,0006 mol) $C_8F_{17}SO_3H$ (entsprechend 0,013 g saurer H-Atome je mol des Perfluoralkylendipropylenoxids) gegeben und die Mischung während 7 Stunden unter Rühren am Rückfluß erhitzt. Nach dem Abkühlen werden zunächst 100 cm³ Diethylether und danach 200 cm³ Wasser zugegeben und gut durchgeschüttelt. Es bilden sich zwei Phasen, die voneinander getrennt werden. Die Ether enthaltende Phase wird zweimal mit Wasser gewaschen, danach mit Natriumsulfat getrocknet und am Rotationsverdampfer der Ether entfernt. Es bleibt ein fester,cremefarbener Rückstand, der noch einige Stunden bei einem Vakuum von 1,33 Pa getrocknet wird. Es werden 16,1 g eines etwas klebrigen Feststoffs erhalten, der nach Untersuchung durch Gelpermeations-Chromatographie zu 93 % aus der Verbindung $CH_2OH-CHOH-CH_2-(CF_2)_6-CH_2-CHOH-CH_2OH$ besteht. An dem Produkt werden folgende Werte gemessen: (OH-Zahl gefunden: 464, berechnet: 498,8). $^{13}C$-NMR (in $CDCl_3$): $CH_2$ 34,4 ppm (Triplett), CHOH 65,9 ppm, $CH_2OH$ 66,2 ppm; Ausbeute 76,7 %.

Beispiele 4 bis 10:

Es wird verfahren wie in Beispiel 1 beschrieben, mit folgenden Unterschieden:
In Beispiel 4 werden als Ausgangsprodukt anstelle des Perfluoroctylpropylenoxids 0,2 mol der Verbindung

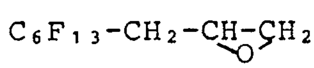

und anstelle des Acetons 350 cm³ 1,4-Dioxan eingesetzt. Anstelle des Amberlyst 15® werden 0,8 g konzentrierter Schwefelsäure zugegeben.
In Beispiel 5 werden anstelle 5 g 10 g Amberlyst 15® zugegeben.
In Beispiel 6 werden anstelle des Acetons 350 cm³ 1,4-Dioxan und die gleiche Menge Amberlyst 15® wie in Beispiel 5 verwendet.
In Beispiel 7 werden anstelle des Acetons 350 ml Ethylenglykoldimethylether eingesetzt.
In Beispiel 8 werden anstelle des Amberlyst 15® 5 g Nafion H® (polymere, fluorierte Verbindung mit $-SO_3H$-Gruppen, die angegebene Menge enthält 0,0048 g saure H-Atome, entsprechend 0,024 g saurer H-Atome je mol des eingesetzten Perfluoralkylpropylenoxids) zugegeben.
In Beispiel 9 werden anstelle Amberlyst 15® 3 g Paratoluolsulfonsäure (entsprechend 0,087 g saurer H-Atome je mol perfluoralkylpropylenoxids)und anstelle des Acetons 350 cm³ 1,4 Dioxan verwendet.
In Beispiel 10 werden anstelle des Amberlyst 15® 1,0 g Perfluoroctylsulfonsäure (entsprechend 0,01 g saurer H-Atome je mol des Perfluoralkylpropylenoxids) und anstelle des Acetons 350 cm³ 1,4-Dioxan eingesetzt. Die, wie vorstehend näher beschrieben, bestimmten Werte sind nachfolgend in einer Tabelle zusammengefaßt.

TABELLE

| Beispiel Nr. | $R_F$- | Säure | g | g saure H-Atome je mol $R_FCH_2CH-CH_2$ \O/ | Lösungsmittel | Rohprodukt Ausbeute (%) | Diolgehalt (%) | OH-Zahl |
|---|---|---|---|---|---|---|---|---|
| 4 * | $C_6F_{13}$ | konz. $H_2SO_4$ | 3,0 | 0,081 | 1,4-Dioxan | 98,4 | 92 | 271 |
| 5 | $C_8F_{17}$ | Amberlyst 15® | 10,0 | 0,205 | Aceton | 97,2 | 92 | 215 |
| 6 | $C_6F_{17}$ | Amberlyst 15® | 10,0 | 0,205 | 1,4-Dioxan | 98,8 | 92 | 220 |
| 7 | $C_8F_{17}$ | Amberlyst 15® | 5,0 | 0,102 | $CH_3OCH_2CH_2OCH_3$ | 96,3 | 92 | 213 |
| 8 | $C_8F_{17}$ | Nafion H® | 5,0 | 0,024 | Aceton | 93,1 | 94 | 215 |
| 9 | $C_8F_{17}$ | p-Toluolsulfonsäure | 3,0 | 0,087 | 1,4-Dioxan | 98,4 | 93 | 215 |
| 10 | $C_8F_{17}$ | $C_8F_{17}SO_3H$ | 1,0 | 0,01 | 1,4-Dioxan | 98,7 | 91 | 211 |

*) Bei der destillativen Reinigung des Rohprodukts aus Beispiel 4 werden 67,8 g $C_6F_{13}-CH_2-CH(OH)-CH_2OH$ erhalten (weißer Feststoff; Kp.: 110 bis 115 °C/106 Pa; Fp. 68 bis 69 °C; Diolgehalt nach GC >98 %; OH-Zahl: gefunden: 281, berechnet: 284,7). Ausbeute: 86,0 %.

Vergleichsversuch A (analog Beispiel 1 der DE-AS 1 568378)

In einem 2000 $cm^3$ Vierhalskolben, der mit Rückflußkühler, Thermometer, Rührer und Tropftrichter versehen ist, werden 285 g (0,6 mol) der Verbindung

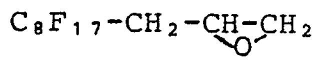

auf 70 bis 80 °C erwärmt. Innerhalb einer Stunde werden 1150 g 30 gew.-%ige Schwefelsäure (entsprechend 11,73 g saurer H-Atome je mol des Perfluoralkylpropylenoxids) unter Rühren zugetropft, wobei nach 15 min eine leicht exotherme Reaktion unter Schäumen einsetzt. Nach Zugabe der Schwefelsäure wird noch 3 h unter Rückfluß gerührt und dann wird die Mischung mit 1500 cm³ Wasser verdünnt. Das abgeschiedene organische Produkt wird abfiltriert, dreimal mit je 1000 cm³ Wasser gewaschen und im Vakuum getrocknet. Es werden 286,7 g eines gelblichen Feststoffs erhalten, an dem folgende Werte gemessen werden: Fp 95 bis 110 °C; OH-Zahl 130; Diol-Gehalt nach Gelpermeations-Chromatographie (HPSEC) 43,2 %. Aus dem Rohprodukt werden durch Vakuumdestillation 114,7 g der reinen Verbindung der Formel $C_8F_{17}CH_2$-CHOH-$CH_2OH$ erhalten (38,7 % Ausbeute). Der Destillationsrückstand besteht nach $^{13}C$-NMR- und Infrarot-Analyse aus einem Gemisch von Etheralkoholen, dessen mittlere Molmasse (bestimmt durch ebullioskopische Messung) ca. 2,3mal höher als die Molmasse der Verbindung $C_8F_{17}CH_2$-CHOH-$CH_2OH$ ist.

Vergleichsversuch B (analog JP Kokai Sho-53-084909)

Eine Mischung aus 47 g (0,1 mol) der Verbindung der Formel

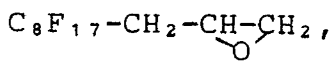

10,0 g (0,17 mol entsprechend 1,7 g saurer H-Atome je mol des Perfluoralkylpropylenoxids) Essigsäure und 0,5 g (0,005 mol) Triethylamin werden 6 h bei 100 °C gerührt. Nach Abkühlen wird die Lösung mit 31,0 g (0,97 mol) Methanol und 0,5 g (0,009 mol) KOH 1 h gerührt und nach Zugabe von 150 cm³ einer 16 gew.-%igen Schwefelsäure noch eine weitere Stunde gerührt. Der abgeschiedene Feststoff wird abgesaugt und mit Wasser neutral gewaschen. Dieses Rohprodukt wird im Vakuum getrocknet und dann destilliert. Am destillierten Produkt werden folgende Werte bestimmt: Reinheit nach Gaschromatographie 97 %; OH-Zahl 223; das $^{13}C$-NMR-Spektrum des so erhaltenen Perfluoralkylpropandiols war mit dem Spektrum des gemäß vorstehend beschriebenen Beispiels 1 hergestellten Produktes identisch. Ausbeute 42,4 g (85,8 %).

**Patentansprüche**

**1.** Verfahren zur Herstellung von Verbindungen der Formel

$ZR_F$-$CH_2$-CHOH-$CH_2OH$ (I)

worin bedeuten

$R_F$ einen geradkettigen, perfluorierten Alkylenrest mit 2 bis 14 C-Atomen oder in Bezug auf die Verbindung der Formel (I) verzweigten, perfluorierten Alkylenrest mit 3 bis 14 C-Atomen,

Z Fluor, Wasserstoff, Chlor oder einen Rest der Formel -$CH_2$-CHOH-$CH_2OH$

durch Umsetzung einer Verbindung der Formel

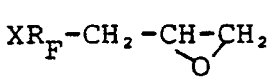

(II)

worin

$R_F$ wie oben definiert ist und

X Fluor, Wasserstoff, Chlor oder einen Rest der Formel

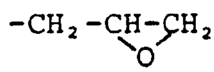

bedeutet,

in Gegenwart von Wasser und einer Säure bei 20 bis 200 °C, gegebenenfalls unter Druck, dadurch gekennzeichnet, daß aus der Verbindung der Formel (II) und mindestens 1 mol Wasser je mol

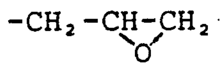

-Gruppe in der Verbindung der Formel (II) sowie mindestens einem nicht reagierenden organischen Lösungsmittel aus der Gruppe Ketone, Ether, Sulfoxide, Sulfone eine unter den Reaktionsbedingungen flüssige homogene Lösung hergestellt wird und diese Lösung umgesetzt wird in Gegenwart von mindestens einer monomeren oder polymeren Säure, die einen $pK_S$-Wert bei 25 °C von höchstens +2 und mindestens eine der folgenden Gruppen $-OSO_3H$,

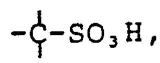

-COOH aufweist, in einer Menge, die 0,001 bis 0,5 g saure H-Atome je 1 mol der Verbindung (II) enthält.

2. Verfahren nach Anspruch 1, dadurch gekennzeichnet, daß eine in der Reaktionsmischung unter den Reaktionsbedingungen weitgehend unlösliche Säure verwendet wird.

3. Verfahren nach einem der Ansprüche 1 oder 2, dadurch gekennzeichnet, daß die Säure eine oder mehrere Arylsulfongruppen aufweist.

4. Verfahren nach einem der Ansprüche 1 oder 2, dadurch gekennzeichnet, daß die Säure eine oder mehrere, gegebenenfalls polymerisierte,Perfluoralkylgruppen aufweist.

5. Verfahren nach einem oder mehreren der Ansprüche 1 bis 4, dadurch gekennzeichnet, daß die Umsetzung durchgeführt wird in Gegenwart von mindestens einer polymeren Säure in einer Menge, die 0,01 bis 0,5 g saure H-Atome je 1 mol der Verbindung (II) enthält.

6. Verfahren nach einem oder mehreren der Ansprüche 1 bis 4, dadurch gekennzeichnet, daß die Umsetzung durchgeführt wird in Gegenwart mindestens einer monomeren, mindestens eine $-OSO_3H$-Gruppe oder

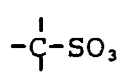

H-Gruppe aufweisenden Säure in einer Menge, die 0,002 bis 0,1 g saure H-Atome je 1 mol der Verbindung (II) enthält.

7. Verfahren nach einem oder mehreren der Ansprüche 1 bis 6, dadurch gekennzeichnet, daß eine Menge des nichtreagierenden organischen Lösungsmittels verwendet wird, die ausreicht, bereits bei Raumtemperatur eine homogene, flüssige Lösung der Verbindung der Formel (II) und des Wassers zu bilden.

8. Verfahren nach einem oder mehreren der Ansprüche 1 bis 7, dadurch gekennzeichnet, daß das oder die nicht-reagierenden organischen Lösungsmittel unter normalem Atmosphärendruck bei 50 bis 200 °C sieden.

## Claims

1. A process for the preparation of compounds of the formula

$ZR_F-CH_2-CHOH-CH_2OH$ (I)

in which

$R_F$ denotes a linear, perfluorinated alkylene radical having 2 to 14 carbon atoms or, in relation to the compound of the formula (I), a branched, perfluorinated alkylene radical having 3 to 14 carbon atoms and

Z denotes fluorine, hydrogen, chlorine or a radical of the formula $-CH_2-CHOH-CH_2OH$,

by reacting a compound of the formula

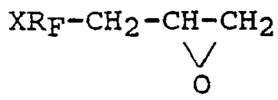

(II)

in which

$R_F$ is as defined above and

X denotes fluorine, hydrogen, chlorine or a radical of the formula

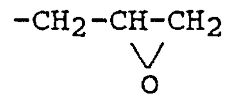

in the presence of water and an acid at 20 to 200 °C, if appropriate under pressure, characterised by preparing a homogeneous solution which is liquid under the conditions of the reaction from the compound of the formula (II) and at least 1 mole of water per mole of

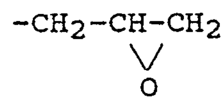

group in the compound of the formula (II) and at least one organic solvent which does not react and belongs to the group comprising ketones, ethers, sulfoxides or sulfones, and reacting this solution in the presence of at least one monomeric or polymeric acid which has a $pK_a$ value at 25 °C of not more than +2 and contains at least one of the following groups: $-OSO_3H$,

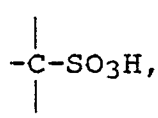

or -COOH, in an amount containing 0.001 to 0.5 g of acid H atoms per mole of the compound (II).

2. The process as claimed in claim 1, characterised in that an acid which is substantially insoluble in the reaction mixture under the reaction conditions is used.

3. The process as claimed in either of claims 1 or 2, characterised in that the acid contains one or more arylsulfonyl groups.

4. The process as claimed in either of claims 1 or 2, characterised in that the acid contains one or more, optionally polymerized, perfluoroalkyl groups.

5. The process as claimed in one or more of claims 1 to 4, characterised in that the reaction is carried out in the presence of at least one polymeric acid in an amount which contains 0.01 to 0.5 g of acid H atoms per mole of the compound (II).

6. The process as claimed in one or more of claims 1 to 4, characterised in that the reaction is carried out in the presence of at least one monomeric acid containing at least one $-OSO_3H$ group or

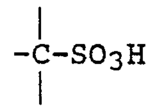

group in an amount which contains 0.002 to 0.1 g of acid H atoms per mole of the compound (II).

7. The process as claimed in one or more of claims 1 to 6, characterised in that an amount of the non-reacting organic solvent which is sufficient, even at room temperature, to form a homogeneous, liquid solution of the compound of the formula (II) and water, is used.

8. The process as claimed in one or more of claims 1 to 7, characterised in that the non-reacting organic solvent(s) boil(s) at 50 to 200 °C under normal atmospheric pressure.

**Revendications**

1. Procédé de préparation de composés de formule

$ZR_F-CH_2-CHOH-CH_2\ OH$ (I)

(dans laquelle :

$R_F$ désigne un radical alkylène linéaire perfluoré en $C_2$ à $C_{14}$ ou bien, par rapport au composé de formule I, un alkylène ramifié perfluoré en $C_3$ à $C_{14}$, et

Z désigne le fluor, l'hydrogène, le chlore, ou un radical $-CH_2-CHOH-CH_2OH$)

par réaction d'un composé de formule

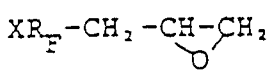 (II)

($R_F$ ayant la signification ci-dessus et

X désignant le fluor, l'hydrogène, le chlore ou un radical

$-CH_2\ -CH-CH_2)$
\o/

en présence d'eau et d'un acide, entre 20 et 200 °C et le cas échéant sous pression, procédé caractérisé en ce que avec le composé de formule II et au moins 1 mol d'eau par moi du groupe

$-CH_2-CH-CH_2$
\o/

du composé de formule II, ainsi qu'un ou plusieurs solvants organiques non réactifs pris parmi des cétones, éthers, sulfoxydes et sulfones, on forme une solution homogène liquide dans les conditions de la réaction, solution que l'on fait réagir en présence d'un ou plusieurs acides monomères ou polymères ayant un $pK_A$ d'au maximum +2 à la température de 25 °C et l'un au moins des groupes $-OSO_3H$,

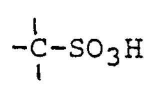

et -COOH, dans une proportion contenant de 0,001 à 0,5 g d'atomes d'hydrogène acides par mol du composé (II).

2. Procédé suivant la revendication 1, caractérisé en ce que l'on opère avec un acide qui est largement

insoluble dans le mélange de réaction dans les conditions réactionnelles.

3. Procédé selon la revendication 1 ou 2, caractérisé en ce que l'acide employé a un ou plusieurs groupes arylsulfoniques.

4. Procédé selon l'une des revendications 1 et 2, caractérisé en ce que l'acide employé a un ou plusieurs groupes perfluoralkyliques éventuellement polymérisés.

5. Procédé selon une ou plusieurs des revendications 1 à 4, caractérisé en ce que l'on effectue la réaction en présence d'un ou de plusieurs acides polymères dans une proportion comprenant de 0,01 à 0,5 g d'atomes H acides par mol du composé (II).

6. Procédé selon une ou plusieurs des revendications 1 à 4, caractérisé en ce que l'on effectue la réaction en présence d'un ou de plusieurs acides monomères ayant au moins un groupe $-OSO_3H$ ou

$$-\overset{|}{\underset{|}{C}}-SO_3H,$$

dans une proportion comprenant de 0,002 à 0,1 g d'atomes H acides par mol du composé (II).

7. Procédé selon une ou plusieurs des revendications 1 à 6, caractérisé en ce que l'on utilise une proportion du solvant organique non réactif suffisante pour former déjà à la température ambiante une solution homogène liquide du composé de formule (II) et de l'eau employée.

8. Procédé selon une ou plusieurs des revendications 1 à 7, caractérisé en ce que le ou les solvants organiques non réactifs ont un point d'ébullition de 50 à 200 °C à la pression atmosphérique normale.